# EUROPÄISCHE PATENTSCHRIFT

(11) **EP 1 051 170 B1**
(45) Veröffentlichungstag und Bekanntmachung des Hinweises auf die Patenterteilung: **10.10.2001**
(21) Anmeldenummer: 98961174.4
(22) Anmeldetag: 11.11.1998
(51) Int. Cl.: A61K 31/35, A61K 31/425

(54) **VERWENDUNG VON SUBSTITUIERTEN AMINOMETHYL-CHROMANEN ZUR VERHINDERUNG DER NEURONALEN DEGENERATION UND ZUR FÖRDERUNG DER NEURONALEN REGENERATION**
APPLICATION OF SUBSTITUTED AMINOMETHYL CHROMANS IN ORDER TO PREVENT NEURAL DEGENERATION AND TO PROMOTE NEURAL REGENERATION
UTILISATION D'AMINOMETHYL-CHROMANES POUR EMPECHER LA DEGENERESCENCE NEURONALE ET FAVORISER LA REGENERATION NEURONALE

(30) Priorität: 24.11.1997 DE 19751949
(43) Veröffentlichungstag der Anmeldung: 15.11.2000
(73) Patentinhaber: Bayer Aktiengesellschaft, 51368 Leverkusen (DE)
(72) Erfinder: FAHRIG, Thomas, D-51429 Bergisch Gladbach (DE); GERLACH, Irene, D-50935 Köln (DE); HORVATH, Ervin, D-51373 Leverkusen (DE); JORK, Reinhard, D-51491 Overath (DE)
(86) Internationale Anmeldenummer: EP9807197
(87) Internationale Veröffentlichungsnummer: WO9926621

(56) Entgegenhaltungen:
- EP-A- 0 749 970
- DE-A- 4 135 474
- US-A- 5 468 753

## Beschreibung

Die Erfindung betrifft die Verwendung von substituierten Aminomethyl-Chromanen zur Herstellung von Arzneimitteln, zur Verhinderung der Degeneration von Nervenzellen (Neurodegeneration) und zur Förderung der neuronalen Regeneration (Neuroregeneration) bei bestimmten cerebralen Verletzungen oder chronischen Erkrankungen des Nervensystems.

Das Nervensystem der Säugetiere besteht im wesentlichen aus zwei unterschiedlichen Zellklassen: (a) den Nervenzellen (Neuronen) und (b) den Gliazellen, die ihrerseits wieder in Oligodendrozyten, Schwannzellen, Mikroglia und Astrozyten unterteilt werden.

Nach jeder Störung der Integrität des Nervensystems reagieren Astrozyten in einer stereotypen Art und Weise, die als reaktive Astrogliose bezeichnet wird. Diese gliale Antwort kann durch eine Reihe verschiedener Verletzungen oder Erkrankungen ausgelöst werden, wie z.B. chirurgische Eingriffe, traumatische, immunologische, chemische, oder ischämische Verletzungen oder neurologische Erkrankungen, wie der Alzheimer'sche Krankheit oder Parkinson'sche Krankheit. Die reaktive Gliose ist durch die Proliferation und Hypertrophie der Zellkörper und cytoplasmatischen Fortsätze von Astrozyten gekennzeichnet. Mit der Reaktion der Astrozyten wird die Expression des Astrozyten-spezifischen Bestandteils des Zellskeletts, glial fibrillary acidic protein (GFAP), verstärkt. Während späterer Phasen stellt GFAP den Hauptbestandteil des gliotischen Narbengewebes dar, das aus der glialen Reaktion resultiert. Gegenwärtig ist die verstärkte Expression von GFAP das einzige konsistente Kennzeichen der reaktiven Gliose.

Die Bildung und Persistenz von glialem Narbengewebe scheint ein Haupthindernis für die Regeneration von Nervenzellen zu sein, denn es hemmt die Bildung und das Auswachsen von neuronalen Fortsätzen sowohl *in vitro* als auch *in vivo* (Reier and Houle, in *Advances in Neurology, Vol. 47: Functional Recovery in Neurological Diseases*, Raven Press, New York [1988], Seiten 87-138). Die Inhibition der Bildung der glialen Narbe zur therapeutischen Behandlung verschiedener neurodegenerativer und neurologischer Erkrankungen könnte deshalb ein neues therapeutisches Prinzip darstellen.

Überraschenderweise zeigte sich nun, daß Aminomethyl-Chromane die GFAP Expression vermindern können. Die durchgeführten Versuche erfolgten in Tieren, deren mittlere Cerebralarterie (MCA) okkludiert wurde und die als Tiermodell des Schlaganfalls Verwendung finden. Diese Versuche deuten darauf hin, daß Aminomethyl-Chromane die Bildung glialen Narbengewebes in vivo vermindern können und damit therapeutisch bedeutend sein können zur Behandlung neurodegenerativer Erkrankungen, die durch die Ausbildung glialen Narbengewebes oder durch reaktive Gliose gekennzeichnet sind, wie beispielsweise die Parkinson'sche Erkrankung, die Amyotrophe Lateralsklerose oder Rückenmarkserkrankungen und/oder -verletzungen.

EP-A-0 352 613, EP-A-0 540 914 und EP-A-0 749 970 beschreiben Aminomethyl-Chroman-Derivate, die sich zur Prophylaxe, Neuroprotektion und Behandlung cerebraler Infarkte (Apoplexia cerebri) wie Schlaganfall und cerebraler Ischämien eignen.

Die therapeutische Wirksamkeit der dort beschriebenen Verbindungen bezieht sich dabei jedoch auf die Neuroprotektion in der akuten Phase des Krankheitsverlaufes. Die akuten Folgen cerebraler Ischämien, wie sie beispielsweise nach einem Schlaganfall auftreten, werden durch Anwendung von neuroprotektiven Arzneimitteln, die die beschriebenen Aminomethyl-Chromane als pharmakologisch wirksame Bestandteile enthalten, vermindert.

Im Gegensatz dazu war es jedoch eine Aufgabe der vorliegenden Erfindung, regenerative Wirkstoffe zur Verfügung zu stellen, die zur Behandlung der post-akuten Phase cerebraler Verletzungen oder zur Behandlung verschiedener chronischer Erkrankungen des Nervensystems geeignet sind.

Die Aufgabe wird erfindungsgemäß gelöst durch die Verwendung von substituierten Aminomethyl-Chromanen der nachfolgenden Formel (I) 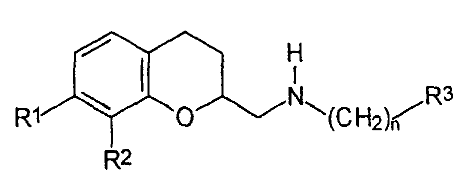 worin
- R¹: für Wasserstoff steht,
- R²: für Wasserstoff, Hydroxy, oder einen Rest der Formel -OCH₃, -OCH₂CH₃, -OCH(CH₃)₂ oder -OCH₂C(CH₃)₂-Cl steht, oder
- R¹ und R²: gemeinsam einen Rest der Formel 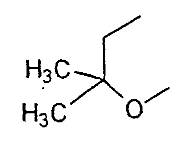 bilden,
- R³: für Cyclopentyl, Cyclohexyl, Cycloheptyl, Cyclooctyl oder den nachfolgenden, als o-Benzosulfimidyl bezeichneten Rest: 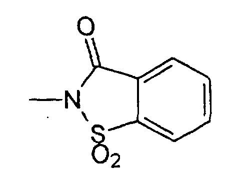 steht und
- n: ausgewählt ist aus 1, 2, 3, 4 oder 5,
sowie deren optische Isomeren und pharmazeutisch annehmbaren Salze,
zur Herstellung eines Medikaments zur Behandlung der Parkinsonschen Erkrankung.

Das Prinzip der Herstellung der erfindungsgemäß verwendeten Aminomethyl-Chromane ist aus der EP-A-0 352 613, der EP-A-0 540 914 oder der EP-A-0 749 970 bekannt.

Im Rahmen der vorliegenden Erfindung können die Verbindungen in verschiedenen stereoisomeren Formen, d. h. in Form ihrer (+)- oder (-)-Enantiomere oder als deren Gemisch (Racemat) vorliegen. Zur Trennung der Racemate in die enantiomeren Formen wird auf die einschlägige, bekannte Fachliteratur verwiesen. Eine bevorzugte Verbindung ist das (-)-Enantiomer der Verbindung der Formel (I) in der R¹ und R² = Wasserstoff, R³ = o-Benzosulfimidyl und n = 4 ist.

Im Rahmen der vorliegenden Erfindung können auch die physiologisch unbedenklichen Salze eingesetzt werden. Physiologisch unbedenkliche Salze der substituierten 2-Aminomethyl-Chromane können Salze der erfindungsgemäßen Verbindungen mit geeigneten organischen oder anorganischen Säuren, insbesondere Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Verbindungen der allgemeinen Formel (I) sowie die sich aus diesen Verbindungen ableitenden pharmazeutische Zusammensetzungen können zur post-akuten therapeutischen Behandlung vielfältiger neurologischer Zustände angewendet werden, bei denen verschiedene Zelltypen des Nervensystems als Folge von neurodegenerativen Erkrankungen oder Eingriffen oder Expositionen degeneriert sind und/oder beschädigt wurden. Insbesondere können Verbindungen der allgemeinen Formel (I) verwendet werden zur Behandlung von Folgezuständen, in denen Schädigungen von Zellen des Nervensystems durch chirurgische Eingriffe, Infektionen, Exposition gegenüber toxischen Agenzien, Tumoren, Ernährungsdefizite oder metabolische Erkrankungen aufgetreten sind. Außerdem können Verbindungen der allgemeinen Formel (I) verwendet werden zur Behandlung der Folgen von neurodegenerativen Erkrankungen, wie der Parkinson'schen Erkrankung, der Multiplen Sklerose, der Amyotrophen Lateralsklerose, der Epilepsie, Drogenmißbrauch oder Drogensucht (Alkohol, Kokain, Heroin, Amphetamin oder ähnliche), Rückenmarkserkrankungen und/oder -verletzungen, Dystrophie oder Degeneration der neuralen Retina (Retinopathien) und peripheren Neuropathien, wie der diabetischen Neuropathie und/oder der durch Toxine induzierte peripheren Neuropathien. Außerdem können Verbindungen der allgemeinen Formel (I) in Verbindung mit chirurgischen Implantationen von Geweben und/oder Prothesen zur Behandlung der neurologischer Erkrankungen und/oder Fehlfunktionen, bei denen eine Implantation angezeigt ist, verwendet werden.

Bevorzugt im Rahmen der Erfindung sind Verbindungen der allgemeinen Formel (I),
wobei
- R¹: für Wasserstoff steht,
- R²: für Wasserstoff, Hydroxy, oder einen Rest der Formel -OCH₃, -OCH(CH₃)₂ oder -OCH₂C(CH₃)₂-Cl steht, oder
- R¹ und R²: gemeinsam einen Rest der Formel 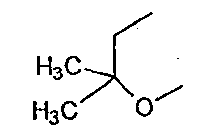 bilden,
- R³: für o-Benzosulfimidyl steht,
- n =: 3 oder 4 ist;
und Aminomethyl-Chromane der allgemeinen Formel (I)
in welcher
- R¹: für Wasserstoff steht,
- R²: für Wasserstoff, Hydroxy, oder einen Rest der Formel -OCH₃ oder -OCH(CH₃)₂ steht, oder
- R¹ und R²: gemeinsam einen Rest der Formel 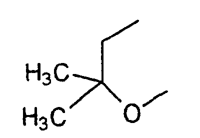 bilden,
- n=: 1 ist und
- R³: für Cyclohexyl oder Cycloheptyl steht.

Besonders bevorzugt sind Verbindungen der allgemeinen Formel (I),
wobei
- R¹: für Wasserstoff steht, und
- R²: für Wasserstoff oder einen Rest der Formel -OCH₃, -OCH(CH₃)₂ oder -OCH₂C(CH₃)₂-Cl steht, oder
- R¹ und R²: gemeinsam einen Rest der Formel 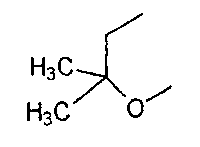 bilden,
- R³: für o-Benzosulfimidyl steht, und
- n=: 4 ist;
und Aminomethyl-Chromane der allgemeinen Formel (I),
in welcher
- R¹: für Wasserstoff steht,
- R²: für Wasserstoff oder -OCH₃ steht,
- n=: 1 ist,
und
- R³: für Cyclopentyl steht.

Allgemein ist bevorzugt, daß, wenn R³ = o-Benzosulfimidyl, n = 3, 4 oder 5, besonders bevorzugt 3 oder 4 ist.

Die Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,1 bis 95 Gew.-%, bevorzugt von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermittel und/oder Dispergiermitteln, wobei z.B. im Fall der Benutzung von Wasser als Verdünnungsmittel gegebenenfalls organische Lösungsmittel als Hilfslösungsmittel verwendet werden können.

Die Hilfsstoffe können beispielsweise ausgewählt sein aus der Gruppe umfassend Wasser, nicht-toxische organische Lösungsmittel, wie Paraffine (z.B. Erdölfraktionen), pflanzliche Öle (z.B. Erdnuß/Sesamöl), Alkohole (z.B. Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumsulfat).

Die Applikation erfolgt in üblicher Weise, vorzugsweise oral oder subcutan, insbesondere intramuskulär oder intravenös. Im Falle der oralen Anwendung können Tabletten selbstverständlich außer den genannten Trägerstoffen auch Zusätze, wie Natriumcitrat, Calciumcarbonat oder Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den oben genannten Hilfsstoffen mit verschiedenen Geschmacksaufbesserern oder Farbstoffen versetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht je 24 Stunden zur Erzielung wirksamer Ergebnisse zu verabreichen. Bei oraler Applikation beträgt die Dosierung etwa 0,01 bis 20 mg/kg, vorzugsweise 0,1 bis 10 mg/kg Körpergewicht je 24 Stunden. Die Verabreichung kann jeweils in Form von Einzelgaben erfolgen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen

Die Erfindung wird durch das nachfolgende Beispiel näher illustriert.

### Beispiel:

Im vorliegenden Beispiel wurde die Testsubstanz während der akuten Phase des Verletzungsgeschehens appliziert, um deren optimale Wirkung zu erzielen. Bewertet wurde jedoch die Substanzwirkung auf die chronische Phase des Krankheitsverlaufs, so daß die Ergebnisse durchaus auf das Potential der Testsubstanz zur Behandlung chronischer Schädigungen hinweisen.

### Okklusion der mittleren Cerebral-Arterie (MCA-O)

Eine unilaterale zerebrale Ischämie wurde an Tribromethanol-narkotisierten Mäusen durch permanenten Verschluß der mittleren Cerebral-Arterie (MCA) induziert. Die Operation erfolgte entsprechend bekannter Methoden (Welsh et al., *J*. *Neurochem.* 49, Seiten 846-851 [1987]) und führt zu einem Infarkt kortikaler und subkortikaler Regionen der ipsilateralen zerebralen Hemisphäre, die durch die linke MCA versorgt wird.

### Bestimmung der GFAP-Immunoreaktivität

Sieben Tage nach Operation wurden die Tiere durch Dekapitation getötet, die Gehirne entnommen, Proteinfraktionen hergestellt und die GFAP-Immunoreaktivität in der "löslichen" Proteinfraktion wie beschrieben bestimmt (Fahrig, *J. Neurochem*. 63, Seiten 1796-1801 [1994]). Der ermittelte GFAP-Gehalt der kontralateralen zerebralen Hemisphäre wurde gleich 100% gesetzt (Kontrolle) und der GFAP-Gehalt der ipsilateralen zerebralen Hemisphäre (d.h. die Hemisphäre, die den Infarktbereich einschließt) wurde in Relation dazu berechnet.

### Behandlung mit Testsubstanz

In diesem Beispiel wurde das (-) Enantiomer der Verbindung der allgemeinen Formel (I) verwendet, mit R¹ und R² = Wasserstoff, R³ = o-Benzosulfimidyl sowie n = 4. Die Verbindung wurde in einer Citrat-gepufferten (Zitronensäure/Natriumcitrat) physiologischen Kochsalzlösung gelöst und durch mehrfache i.v. Injektionen unmittelbar, 2 und 4 Stunden nach der Operation appliziert. Unter diesen Bedingungen reduzierte die Verbindung die Ischämie-induzierte GFAP-Immunoreaktivität (und damit die gliale Narbenbildung) Dosis-abhängig (Tabelle 1).

**Tabelle 1:**

| *Reduktion der GFAP-Immunoreaktivität durch ein Aminomethyl-Chro- man der allgemeinen Formel (I) mit R*^{*1*} *und R*^{*2*} = *H*, *R*^{*3*} *= o-Benzosulfimidyl, n = 4* | | | | |
|---|---|---|---|---|
| **Dosis** | **1 µg/kg** | **10 µg/kg** | **30 µg/kg** | **100 µg/kg** |
| **GFAP Immunoreaktivität [% der Kontrolle]** | 94.0 | 79.7 | 62.3 | 59.5 |
| **S.E.M. *[%]** | 3.2 | 9.2 | 8.5 | 3.4 |
| **Reduktion der GFAP Immuno- reaktivität [%]** | - 6.0 | - 20.3 | - 37.7 | - 40.5 |

| | | | | |
|---|---|---|---|---|
| * Standardfehler des Mittelwertes | | | | |

## Patentansprüche

1. Verwendung von substituierten Aminomethyl-Chromanen der nachfolgenden Formel (I), 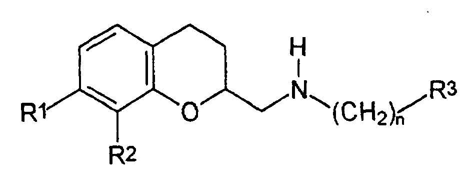 worin
R¹ für Wasserstoff steht,
R² für Wasserstoff, Hydroxy, oder einen Rest der Formel -OCH₃, -OCH₂CH₃ , -OCH(CH₃)₂ oder -OCH₂C(CH₃)₂-Cl steht, oder
R¹ und R² gemeinsam einen Rest der Formel 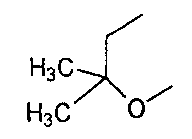 bilden,
R³ für Cyclopentyl, Cyclohexyl, Cycloheptyl, Cyclooctyl oder o-Benzosulfimidyl steht und
n ausgewählt ist aus 1, 2, 3, 4 oder 5,
sowie deren optische Isomeren und pharmazeutisch annehmbaren Salze, zur Herstellung eines Medikaments zur Behandlung der Parkinsonschen Erkrankung.

2. Verwendung gemäß Anspruch 1, **dadurch gekennzeichnet, daß** in Formel (I), n = 3, 4 oder 5 und R³ = o-Benzosulfimidyl ist.

3. Verwendung gemäß Anspruch 1 oder 2, **dadurch gekennzeichnet, daß** in Formel (I)
R¹ für Wasserstoff steht,
R² für Wasserstoff, oder einen Rest der Formel -OCH₃, -OCH(CH₃)₂ oder -OCH₂C(CH₃)₂-Cl steht, oder
R¹ und R² gemeinsam einen Rest der Formel 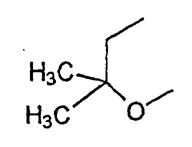 bilden,
R³ = o-Benzosulfimidyl und n = 4 ist.

4. Verwendung gemäß Anspruch 1, **dadurch gekennzeichnet, daß** in Formel (I)
R¹ für Wasserstoff steht,
R² für Wasserstoff, -OCH₃ oder -OCH(CH₃)₂ steht, oder
R¹ und R² gemeinsam einen Rest der Formel 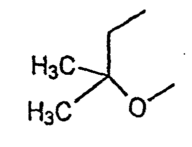 bilden,
R³ für Cyclohexyl oder Cycloheptyl steht und
n= 1 ist.

5. Verwendung gemäß Anspruch 4, **dadurch gekennzeichnet, daß** in Formel (I)
R¹ für Wasserstoff steht,
R² für Wasserstoff oder -OCH₃ steht,
R³ für Cycloheptyl steht und
n = 1 ist.

6. Verwendung gemäß Anspruch 1, **dadurch gekennzeichnet, daß** in Formel (I)
R¹ und R² für Wasserstoff stehen,
R³ für o-Benzosulfimidyl und n für 4 steht.

7. Verwendung nach irgendeinem der Ansprüche 1 bis 6, **dadurch gekennzeichnet, daß** die Verbindungen der Formel (I) die (-)-Enantiomerkonfiguration aufweisen.

8. Verwendung von Verbindungen, welche die in einem der Ansprüche 1 bis 7 angegebene Bedeutung haben, zur herstellung eines Medikamentes zur regenerativen Behandlung von neurologischen Zuständen, welche die Folgen sind von Schädigungen durch chirurgische Eingriffe, Infektionen, Implantationen, Exposition gegenüber toxischen Agenzien, Tumoren, Ernährungsdefiziten oder metabolischen Erkrankungen, Multipler Sklerose, Amyotropher Lateralsklerose, Epilepsie, Drogenmißbrauch oder Drogensucht, Rückenmarkserkrankungen und/oder -verletzungen, Dystrophie oder Degeneration der neuralen Retina oderperipheren Neuropathien.

## Claims

1. Use of substituted aminomethyl-chromans of the following formula (I) 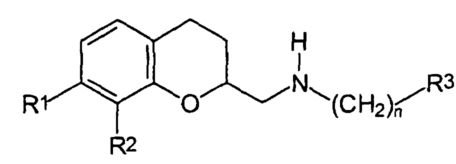 in which
R¹ represents hydrogen,
R² represents hydrogen, hydroxyl or a radical of the formula -OCH₃, -OCH₂CH₃, -OCH(CH₃)₂ or -OCH₂C(CH₃)₂-Cl or
R¹ and R² together form a radical of the formula 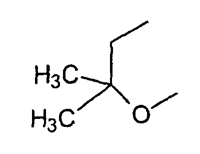
R³ represents cyclopentyl, cyclohexyl, cycloheptyl, cyclooctyl or o-benzenesulphimidyl and
n is selected from 1, 2, 3, 4 or 5,
and their optical isomers and pharmaceutically acceptable salts,
for the production of a medicament for the treatment of Parkinson's disease.

2. Use according to Claim 1, **characterized in that**, in formula (I), n = 3, 4 or 5 and R³ = o-benzenesulphimidyl.

3. Use according to Claim 1 or 2, **characterized in that**, in formula (I),
R¹ represents hydrogen,
R² represents hydrogen or a radical of the formula -OCH₃, -OCH(CH₃)₂ or -OCH₂C(CH₃)₂-Cl, or
R¹ and R² together form a radical of the formula 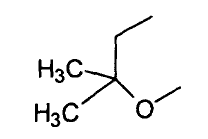
R³ = o-benzenesulphimidyl and n = 4.

4. Use according to Claim 1, **characterized in that**, in formula (I),
R¹ represents hydrogen,
R² represents hydrogen, -OCH₃ or -OCH(CH₃)₂, or
R¹ and R² together form a radical of the formula 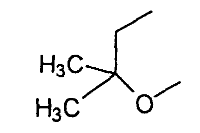
R³ represents cyclohexyl or cycloheptyl and
n= 1.

5. Use according to Claim 4, **characterized in that**, in formula (I),
R¹ represents hydrogen,
R² represents hydrogen or -OCH₃,
R³ represents cycloheptyl and
n = 1.

6. Use according to Claim 1, **characterized in that**, in formula (I),
R¹ and R² represent hydrogen,
R³ represents o-benzenesulphimidyl and n represents 4.

7. Use according to any of Claims 1 to 6, **characterized in that** the compounds of the formula (I) have the (-) enantiomer configuration.

8. Use of compounds which have the meaning given in one of Claims 1 to 7 for the production of a medicament for the regenerative treatment of neurological conditions which are the sequelae of damage due to surgical interventions, infections, implantations, exposure to toxic agents, tumours, nutritional deficits or metabolic disorders, multiple sclerosis, amyotrophic lateral sclerosis, epilepsy, drug abuse or drug addiction, bone marrow disorders and/or injuries, dystrophy or degeneration of the neural retina or peripheral neuropathies.

## Revendications

1. Utilisation d'aminométhyl-chromanes substitués répondant à la formule (I) ci-après 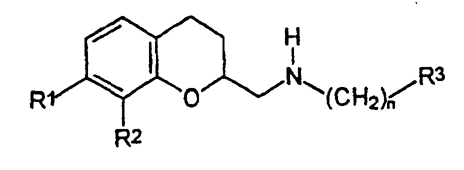 dans laquelle
R¹ représente un atome d'hydrogène
R² représente un atome d'hydrogène, un groupe hydroxyle ou un radical répondant aux formules -OCH₃, -OCH₂CH₃, -OCH(CH₃)₂ ou encore -OCH₂C(CH₃)₂-Cl, ou bien
R¹ et R² forment ensemble un radical répondant à la formule 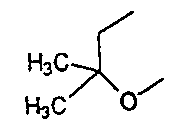
R³ représente un groupe cyclopentyle, un groupe cyclohexyle, un groupe cycloheptyle, un groupe cyclooctyle ou un groupe o-benzosulfimidyle, et
n est choisi parmi les nombres 1, 2, 3, 4 ou 5, ainsi que de leurs isomères optiques et de leurs sels pharmaceutiquement acceptables,
pour la préparation d'un médicament pour le traitement de la maladie de Parkinson.

2. Utilisation selon la revendication 1, **caractérisée en ce que**, dans la formule (I), n représente 3, 4 ou 5 et R³ représente un groupe o-benzosulfimidyle.

3. Utilisation selon la revendication 1 ou 2, **caractérisée en ce que**, dans la formule (I),
R¹ représente un atome d'hydrogène
R² représente un atome d'hydrogène ou un radical répondant aux formules -OCH₃, -OCH(CH₃)₂ ou encore -OCH₂C(CH₃)₂-Cl, ou bien
R¹ et R² forment ensemble un radical répondant à la formule 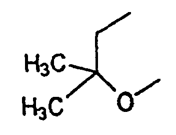
R³ représente un groupe o-benzosulfimidyle, et
n est égal à 4.

4. Utilisation selon la revendication 1, **caractérisée en ce que**, dans la formule (I),
R¹ représente un atome d'hydrogène
R² représente un atome d'hydrogène, un radical -OCH₃ ou un radical -OCH(CH₃)₂ ou bien
R¹ et R² forment ensemble un radical répondant à la formule 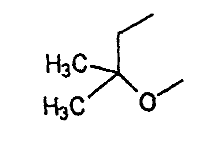
R³ représente un groupe cyclohexyle ou un groupé cycloheptyle, et
n est égal à 1.

5. Utilisation selon la revendication 4, **caractérisée en ce que**, dans la formule (I),
R¹ représente un atome d'hydrogène
R² représente un atome d'hydrogène ou un radical -OCH₃,
R³ représente un groupe cycloheptyle, et
n est égal à 1.

6. Utilisation selon la revendication 1, **caractérisée en ce que**, dans la formule (I),
R¹ et R² représentent un atome d'hydrogène,
R³ représente un groupe o-benzosulfimidyle, et
n est égal à 4.

7. Utilisation selon l'une quelconque des revendications 1 à 6, **caractérisée en ce que** les composés répondant à la formule (I) présente la configuration énantiomère (-).

8. Utilisation de composés, qui possèdent la signification indiquée dans l'une quelconque des revendications 1 à 7, pour la préparation d'un médicament destiné au traitement régénératif d'états neurologiques qui sont les conséquences de dégradations dues à des interventions chirurgicales, à des infections, à des implantations, à une exposition à des agents toxiques, à des tumeurs, à des déficits alimentaires ou à des maladies métaboliques, à la sclérose multiple, à la sclérose latérale amyotrophique, à l'épilepsie, à l'abus de drogues ou à la toxicomanie, à des maladies et/ou à des blessures de la moelle épinière, à une dystrophie ou à une dégénérescence de la rétine neurale, ou encore à des neuropathies périphériques.
